# FASCICULE DE BREVET EUROPEEN

(11) **EP 1 147 410 B1**
(45) Date de publication et mention de la délivrance du brevet: **20.11.2002**
(21) Numéro de dépôt: 99961087.6
(22) Date de dépôt: 15.12.1999
(51) Int. Cl.: G01N 33/08

(54) **Procédé non-destructif et dispositif d'évaluation et de sélection de produits selon leurs qualités**
Unschädliches Verfahren und Vorrichtung zur Auswertung und Selektion von Produkten gemäss ihrer Qualität
Non-destructive method and device for assessment and selection of products according their quality

(30) Priorité: 16.12.1998 FR 9816070
(43) Date de publication de la demande: 24.10.2001
(73) Titulaire: Kalmar, Christian, 67100 Strasbourg (FR)
(72) Inventeur: Kalmar, Christian, 67100 Strasbourg (FR)
(74) Mandataire: Nuss, Pierre
(86) Numéro de dépôt international: FR9903155
(87) Numéro de publication internationale: WO00036411

(56) Documents cités:
- EP-A- 0 602 285
- WO-A-93/17318
- US-A- 4 161 366
- US-A- 5 696 325

## Description

La présente invention concerne le domaine du contrôle de la qualité de produits ovoïdes et plus particulièrement d'oeufs, et a pour objet un nouveau procédé non-destructif d'évaluation et de sélection de produits ou d'objets selon leurs qualités ainsi qu'un dispositif pour la mise en oeuvre de ce procédé.

Avec l'apparition de nouvelles normes de production de plus en plus contraignantes, les contrôles en matière de qualité sont devenus de nos jours incontournables et se multiplient à tous les niveaux dans la plupart des domaines industriels.

Ces contrôles permettent d'assurer une plus grande sécurité au consommateur et lui garantissent un produit de qualité sans cesse améliorée et de plus en plus constante.

De nombreux produits, notamment ceux destinés à l'alimentation ou aux soins de l'homme ou des animaux, doivent satisfaire à des contraintes d'ordres techniques, mais également à des dispositions réglementaires très strictes en matière d'hygiène et de santé. De plus, ces réglementations ont tendance à devenir de plus en plus contraignantes.

Un exemple particulièrement critique est celui de la détection et de l'élimination d'oeufs fêlés, qui vont se fêler ultérieurement, ou qui sont sales (traces de fèces ou de sang sur la coquille pouvant indiquer une maladie bactérienne ou virale de la poule). En effet, certains oeufs endommagés ou malformés présentent dans leurs coquilles des fissures assez profondes pour permettre aux bactéries et autres germes pathogènes de s'y introduire et de s'y développer.

La coquille d'un oeuf n'est en général pas uniforme. Lorsqu'on observe par transparence une coquille d'oeuf, on observe des taches plus ou moins claires ou des lignes transparentes correspondant à des traits que l'on appelle parfois traits de crayon ou coups de griffe. Lorsque deux ou plusieurs de ces lignes se croisent les aviculteurs disent que l'oeuf possède une étoile.

Les oeufs qui ont une étoile dans leurs coquilles ont de fortes chances de se briser pendant le transport. Pour détecter ces oeufs, ceux-ci passent sur une table de mirage où une série de lampes éclairant les oeufs permet leur observation par transparence.

Cependant, ce type de défaut reste difficilement repérable à l'oeil nu, de sorte qu'un contrôle manuel dépend surtout de l'expérience du contrôleur. Il en résulte que ce type de contrôle peut trop facilement devenir aléatoire, fastidieux et onéreux à mettre en oeuvre même pour des petits producteurs. Le risque d'une élimination incomplète des oeufs défectueux par un contrôle manuel peut ainsi être intolérablement élevé.

En ce qui concerne la détection automatisée des micro-fissures dans les coquilles d'oeufs, plusieurs procédés sont actuellement utilisés :

Un premier procédé consiste à faire rouler l'oeuf à tester dans un couloir où un ensemble de générateurs de signaux (transducteurs à ultrasons) met l'oeuf en vibration. A l'opposé de cet ensemble se situent des capteurs (micros à ultrasons) qui détectent les signaux émis. Les signaux sont analysés et un coefficient de qualité est attribué à chaque oeuf testé.

Un autre procédé d'analyse prévoit de frapper l'oeuf mis en rotation sur une bande transporteuse par un ensemble de billes métalliques en lévitation dans un champ magnétique. Chaque bille frappe trois fois l'oeuf. Quatre billes frappent l'oeuf à gauche, puis quatre billes frappent l'oeuf au milieu, puis quatre billes frappent l'oeuf sur le côté droit. Tous ces chocs sont analysés et un coefficient de qualité est attribué à chaque oeuf.

Un troisième procédé utilise un petit laser de puissance pour émettre un rayon de lumière sur l'oeuf à analyser. L'échauffement instantané provoque un petit choc thermique qui se transforme en écho ondulatoire. Le signal est analysé ; un coefficient de qualité est également attribué à chaque oeuf testé.

Tous les procédés automatisés sont basés sur le même principe, à savoir, stimuler l'oeuf par une impulsion ou une série d'impulsions puis enregistrer la réponse oscillatoire de l'oeuf. Toutefois, ceux-ci ne sont pas satisfaisants. En effet, le premier procédé décrit n'est pas assez sensible et fiable dans la mesure où il possède encore une marge d'erreur parfois supérieure à 25 % en ce qui concerne les oeufs défectueux non détectés.

Le second et le troisième ont une bonne sensibilité mais fissurent les oeufs (impacts de billes en acier ou du laser).

De plus, aucun de ces systèmes ne détecte le piquage des oeufs, c'est-à-dire les trous minuscules dus aux coups de bec de poules. Pour détecter le piquage et les oeufs sales, certains producteurs utilisent des caméras pour observer la surface des oeufs. Cependant, ces dispositifs ne détectent pas les micro-fissures et difficilement les petits trous au niveau des pôles.

US-A-4,161,366 décrit un procédé et un appareil d'évaluation automatique des oeufs dans lequel les oeufs sont illuminés et après vibrés méchaniquement. WO-A-93/17318 décrit un procédé non-destructif et un appareil d'évaluation des oeufs, qui est basé sur des mesures de résonance acoustique des oeufs réalisées avec un transducteur piezoélectrique.

Par ailleurs, la qualité et le prix de vente d'un oeuf dépendent également de la qualité de son contenu qui n'est pas non plus facilement analysable.

Il existe donc un besoin de déterminer, d'une manière simple et avec une fiabilité élevée (supérieure à 95 %), la présence de défauts sur les coquilles d'oeuf, notamment la présence de taches, de trous, de micro-fissures, ainsi que la qualité de son contenu de manière, notamment, à éviter les intoxications dues à des oeufs contaminés par des germes. Un tel procédé de détection doit être simple et peu onéreux à mettre en oeuvre et permettre au producteur de garantir une qualité constante de sa production et de vendre ses oeufs au meilleur prix.

La présente invention a pour but de fournir un procédé palliant les inconvénients précités ainsi que le dispositif pour sa mise en oeuvre.

A cet effet, elle a pour objet un procédé non-destructif d'évaluation et de sélection de produits ou objets ovoïdes, notamment des oeufs, selon leurs qualités, caractérisé en ce qu'il comprend les étapes consistant à :
- opérer une série de mesures ondulatoires de manière à obtenir une série de signatures ondulatoires traitées pour fournir une première série de paramètres desdits produits ou objets à analyser,
- opérer une série de mesures optiques de manière à obtenir une série de signatures optiques traitées pour fournir une seconde série de paramètres desdits produits ou objets à analyser,
- corréler lesdites première et seconde séries de paramètres de façon à déterminer un facteur de qualité pour chaque produit ou objet analysé, et,
- différencier les produits ou objets analysés selon les facteurs de qualité obtenus.

L'invention sera mieux comprise, grâce à la description ci-après, qui se rapporte à un mode de réalisation préféré, donné à titre d'exemple non limitatif, et expliqué avec référence aux dessins schématiques annexés, dans lesquels :
- la figure 1 représente une vue de dessus simplifiée de connexion des éléments du dispositif pour la mise en oeuvre du procédé de la présente invention ;
- la figure 2 représente une vue de face du dispositif d'analyse ondulatoire ;
- la figure 3 représente une vue de dessus du dispositif d'analyse ondulatoire ;
- la figure 4 représente une vue en élévation latérale des éléments du dispositif d'analyse optique ;
- la figure 5 représente une courbe de réponse idéalisée d'une mesure ondulatoire pour l'obtention de paramètres relatifs au produit analysé, et ;
- la figure 6 représente une courbe de réponse idéalisée d'une mesure optique pour l'obtention de paramètres relatifs au produit analysé ;

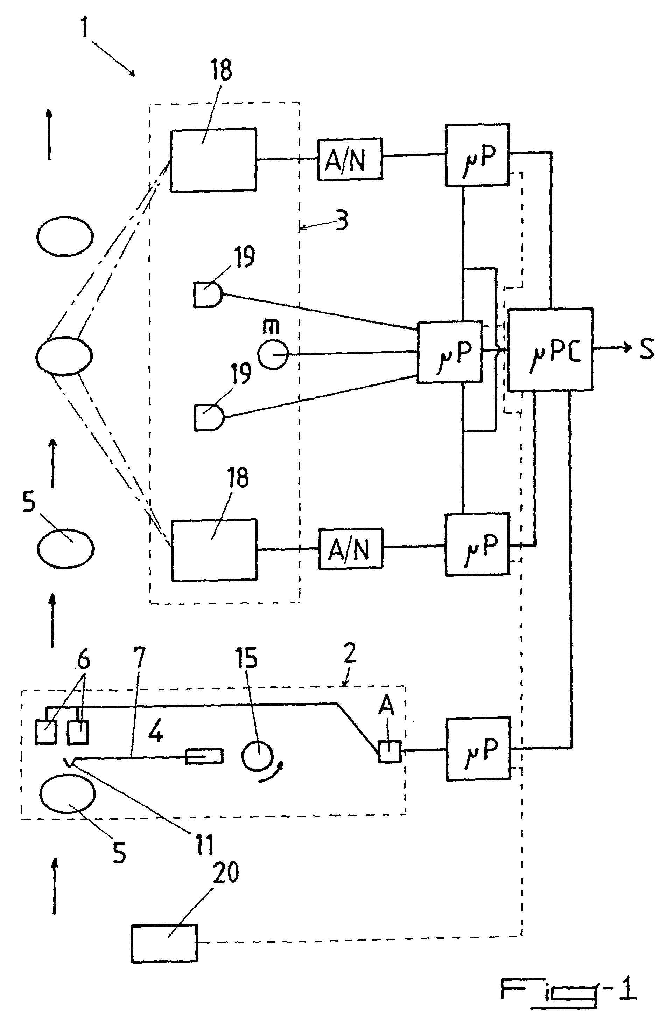

Les figures 1 à 4 montrent, à titre d'exemple non limitatif, un mode de réalisation du procédé et du dispositif 1 pour la mise en oeuvre du procédé, conformes à la présente invention. La figure 1 représente une vue de dessus simplifiée de l'ensemble du dispositif 1 comprenant le dispositif d'analyse ondulatoire 2 et le dispositif d'analyse optique 3 reliés à des microprocesseurs µP et à un microprocesseur central µPC de manière à travailler en synergie ensemble.

Alors que la figure 1 représente une vue de dessus de l'ensemble du dispositif 1, les différents éléments constitutifs de ce dernier, à savoir, les dispositifs d'analyse ondulatoire 2 et le dispositif d'analyse optique 3 sont représentés dans des vues en élévations latérales pour des raisons de clarté.

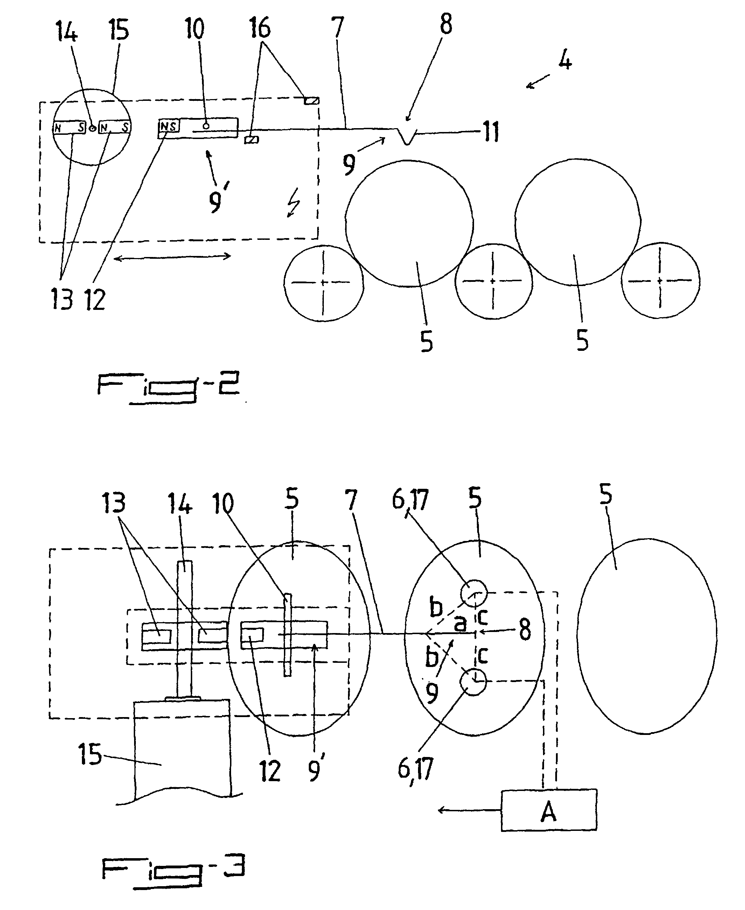

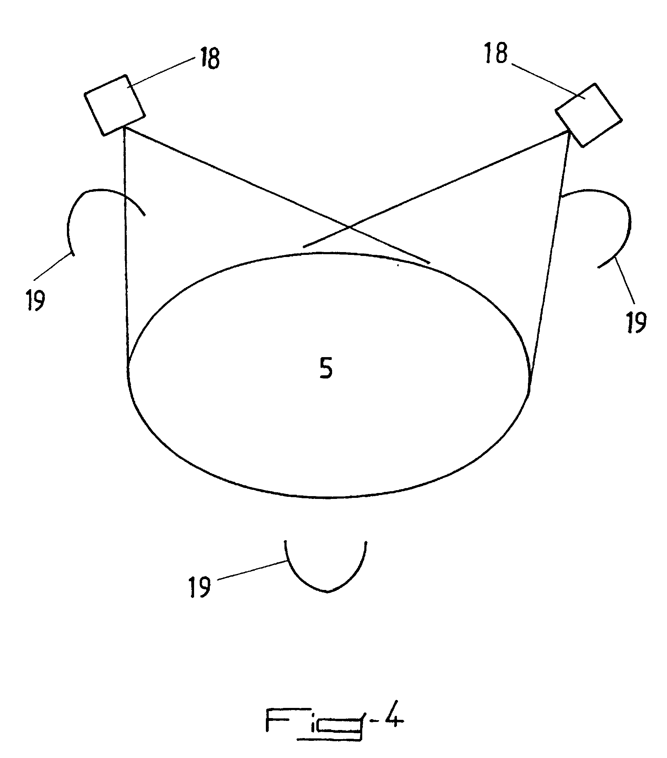

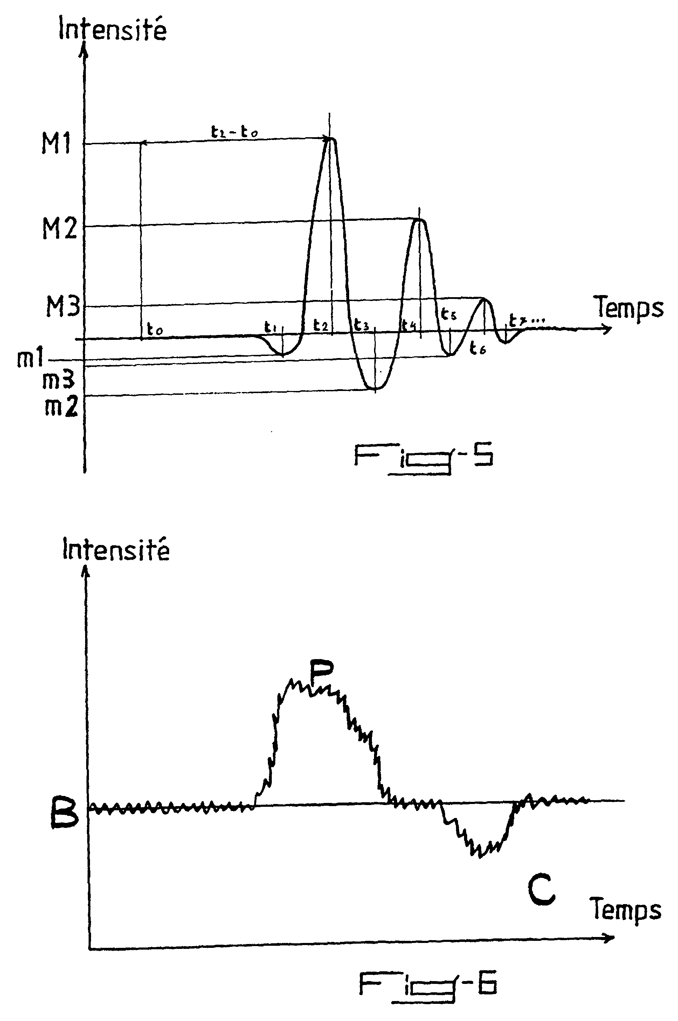

Pour la mesure optique de l'oeuf 5 on se référera à la figure 4 qui montre précisément la disposition préférée des différents éléments dudit dispositif d'analyse optique 3, ceux-ci n'étant présents sur la figure 1 que pour en illustrer le schéma de connexion de principe avec les autres éléments du dispositif 1.

Le procédé selon la présente invention est caractérisé en ce qu'il comprend les étapes consistant à :
- opérer une série de mesures ondulatoires de manière à obtenir une série de signatures ondulatoires traitées pour fournir une première série de paramètres desdits produits ou objets 5 à analyser,
- opérer une série de mesures optiques de manière à obtenir une série de signatures optiques traitées pour fournir une seconde série de paramètres desdits produits ou objets 5 à analyser,
- corréler lesdites première et seconde séries de paramètres de façon à déterminer un facteur de qualité pour chaque produit ou objet 5 analysé, et,
- différencier les produits ou objets 5 analysés selon les facteurs de qualité obtenus.

Bien entendu, le procédé de la présente invention s'applique à tous les produits ou objets 5 ovoïdes dont la qualité est susceptible d'être déterminée de cette façon. De manière préférentielle, le procédé conforme à la présente invention est caractérisé en ce que les produits ou objets 5 à analyser consistent en des oeufs 5, en particulier, en des oeufs 5 de poule. Pour des raisons de simplification, le procédé décrit ci-après sera appliqué au contrôle des oeufs 5 sans qu'il s'agisse là d'une quelconque limitation.

Selon un mode de réalisation, le procédé de la présente invention est caractérisé en ce que les mesures ondulatoires sont effectuées en réalisant une succession d'impacts, à fréquence élevée, à différents endroits de la périphérie ou de la surface extérieure du produit ou de l'objet 5 à analyser et à acquérir les ondes résultantes desdits impacts. A titre d'exemple, la durée d'une mesure pour un oeuf 5 de poule est comprise entre 30 ms et 100 ms, une série comportant une dizaine de mesures durant environ 300 ms.

Pour réaliser les mesures ondulatoires, l'oeuf 5 à analyser peut, par exemple, se déplacer en tournant autour de son axe longitudinal, le dispositif d'analyse ondulatoire 2 suivant l'oeuf 5 à la vitesse d'avancement de ce dernier jusqu'à la réalisation de la dernière mesure. Les mesures ondulatoires se font par l'intermédiaire du dispositif d'analyse ondulatoire 2 décrit plus en détail ci-après. De manière préférentielle, le tracé sur la coquille de l'oeuf 5 analysé des différents cndroits d'impacts des mesures ondulatoires représente un cercle dont tous les points sont à une distance constante de l'un des pôles de l'oeuf 5. De manière essentielle, les distances a, b, c, sur la figure 3 restent constantes pendant que les mesures sont faites sur l'oeuf 5.

La dernière mesure d'une série étant effectuée, le dispositif d'analyse ondulatoire 2 retourne à sa position initiale pour commencer une nouvelle série de mesure sur un autre oeuf 5. Ce retour peut se faire par tout moyen mécanique suffisamment rapide robuste et fiable, par exemple, par l'intermédiaire d'un ensemble du type bielle-manivelle ou d'un ensemble came rotative-ressort de rappel assurant un mouvement de va-et-vient à l'ensemble de mesure. A titre indicatif, et pour une série de mesure durant environ 300 ms, la distance parcourue par l'oeuf 5 et les instruments de mesure est de l'ordre de 3 à 4 cm.

Le dispositif d'analyse ondulatoire 2 suit avec précision l'oeuf 5 sur environ les deux tiers de son parcours, le tiers restant permettant audit dispositif d'analyse ondulatoire 2 de retourner à sa position initiale et de se positionner sur l'oeuf 5 suivant.

La distance moyenne entre deux oeufs 5 consécutifs est de l'ordre de 5 cm et peut varier en fonction de la vitesse et du type de la machine. Dans ce cas, il suffit de modifier le rapport de temps entre l'aller et le retour.

Entre deux séries consécutives de mesures de deux oeufs 5 consécutifs, les résultats des mesures ondulatoires sont transférés à un microprocesseur µP par l'intermédiaire d'un amplificateur A (cf. figure 1). Le microprocesseur µP établit une signature pour chaque échelon. Après un certain nombre d'échelons, il effectue un traitement de la série de signatures obtenue et la transforme en une série de paramètres correspondants (dureté de la coquille, taille, résonance, âge de l'oeuf...).

La courbe de la figure 5 donne un exemple simplifié de l'enregistrement de l'intensité d'une réponse ondulatoire (acoustique) en fonction du temps. Le point de départ t₀ de la courbe correspond du début du basculement du moyen 4 de formation de l'onde de choc. Après le choc et un certain temps de latence, on observe à l'instant t₁ un premier minimum m₁ dont la valeur permet de déduire la résistance de la coquille du produit (de l'oeuf 5) analysé. Le temps t₂-t₀ entre le début du basculement et le premier maximum M₁ permet de déduire la taille de l'oeuf 5 analysé.

Les valeurs des autres extréma (m₂, m₃... ; M₂, M₃...) sont également stockées en mémoire et servent de même à déterminer d'autres paramètres de qualité de l'oeuf 5 testé (principe statistique dit "des cibles" ou "des barycentres" employant la formule de Bayles).

Le paramètres obtenus sont ensuite transférés à un microprocesseur central µPC qui les stocke, si nécessaire, dans une mémoire jusqu'au traitement final permettant, avec les paramètres obtenus par les autres microprocesseurs µP, d'attribuer une note finale de qualité à l'oeuf 5 examiné en fonction de la pondération des différents critères de qualités intermédiaires, laquelle pondération peut être définie de manière empirique ou directement par la saisie des données issues du cahier des charges du producteur.

En ce qui concerne les mesures optiques, celles-ci sont réalisées par une série de relevés de signaux optiques alternativement par transparence et par réflexion. Le dispositif d'analyse optique 3 sera décrit plus en détail ci-après.

La courbe de la figure 6 donne un exemple simplifié de l'enregistrement de l'intensité d'une réponse mesure optique en fonction du temps. Par rapport à la ligne de base B, le pic P représente un maximum (passage) de lumière alors que le creux C correspond à un minimum (absorption) de lumière. Les images obtenues par les dispositifs photonumériques rapides 18 de la figure 1 sont des ensembles de lignes de pixels stockés sous la forme de matrices de pixels (1 pixel = 3 x 8 bits dont 8 pour la couleur rouge, 8 pour la couleur bleue et 8 pour la couleur verte).

Un procédé de seuillage appliqué aux lignes obtenues consiste à analyser chaque ligne, l'une après l'autre, et à déterminer l'existence d'éventuels excédents ou de manques de lumière sur les trois couleurs. Le maximum ou pic P sur la courbe de la figure 6 correspond donc à un défaut favorisant le passage de la lumière à travers la coquille de l'oeuf 5 (trou, micro-fissure...) alors que le minimum ou creux C correspond à un défaut favorisant l'absorption de la lumière (saleté, taches, inclusion...) sur la coquille ou dans l'oeuf 5. En l'absence de tels pics P ou de tels creux C l'oeuf 5 pourra être déclaré "bon".

A la fin du seuillage, on obtient également une valeur moyenne pour les trois couleurs de base ce qui permet de déterminer la couleur de l'oeuf 5 analysé. La couleur de l'oeuf 5 peut être utilisée comme facteur de qualité et donc servir de critère de sélection.

Les lignes analysées individuellement puis regroupées dans une matrice permettent ensuite de déterminer le contour des défauts visibles de l'oeuf 5.

Les mesures optiques étant effectuées deux fois, une fois par réflexion et une fois par transparence, on obtient deux images de la surface complète de l'oeuf 5. Ceci permet notamment de déterminer si une tache présente sur l'une ou les deux images correspond à un défaut qui se situe à l'intérieur ou à l'extérieur de la coquille de l'oeuf 5. On sait en effet, qu'une image restant dans une zone fixe pendant la rotation de l'oeuf 5 au cours de plusieurs mesures successives correspond à une inclusion alors qu'une image mobile correspond à une tache sur la coquille de l'oeuf 5. Pour déterminer si le défaut est à l'extérieur ou à l'intérieur de l'oeuf 5 (salissure externe ou tache de sang dans l'oeuf), on observe la présence de la tache caractérisant le défaut sur les clichés obtenus par réflexion et par transparence. Une tache à la fois présente sur les deux types de clichés signifie que le défaut est une tache externe (salissure sur la coquille) alors qu'une tache de sang dans l'oeuf 5 ne fera pas apparaître de tache sur les clichés obtenus par réflexion.

Les contours de la tache ou zone globale des défauts visibles étant déterminés sur les images obtenues pour l'oeuf 5, on trace à l'intérieur de cette zone, des bandes qui correspondent aux directions principales des différentes sous-zones formant, par regroupement et recoupement, la zone globale des défauts visibles.

On vérifie ensuite si ces bandes se croisent. Dans ce cas l'oeuf 5 aura une probabilité plus élevée de se fendre à cette ou ces intersections, la probabilité d'une fissure de l'oeuf 5 étant proportionnelle au nombre et à la surface des intersections des bandes. Bien entendu, la probabilité de fissuration est un facteur de qualité et donc un critère de sélection important.

L'étape de corrélation des signatures optiques et ondulatoires obtenues consiste à transformer les séries de mesures en paramètres puis à superposer les signaux desdites signatures par l'intermédiaire d'un traitement du signal de manière à obtenir une image résultante pour chaque série de mesures et à déduire le degré de qualité du produit analysé.

En particulier, la corrélation se fait par l'intermédiaire d'un moteur d'inférence 0⁺ qui, en fonction des première et seconde séries de paramètres obtenus (connaissances au sens du moteur d'inférence) et dans les zones d'ambiguïté - c'est-à-dire les zones de transition entre deux niveaux de qualité consécutifs (par exemple entre bon et très bon) - prend une décision quant au niveau de qualité finalement attribué à l'oeuf 5.

De manière avantageuse, la corrélation pourra éventuellement même préciser la nature du défaut de l'oeuf 5 analysé (salissure ou fissure).

La présente invention a également pour objet un dispositif 1 pour la mise en oeuvre du procédé tel que décrit ci-dessus. Un tel dispositif 1 est constitué par:
- au moins un dispositif d'analyse ondulatoire 2,
- au moins un dispositif d'analyse optique 3, et,
- au moins un moyen de traitement informatique des données.

Comme on le voit sur les figures 1 à 3, le dispositif d'analyse ondulatoire 2 conforme à la présente invention comporte un premier moyen 4 de formation d'une onde de choc sur la surface extérieure du produit 5 à analyser ainsi qu'un second moyen 6 d'acquisition de l'onde résultante de l'impact sur ledit produit 5 à analyser (pour plus de clarté le moyen 6 n'a pas été représenté sur la figure 2).

Le moyen 4 de formation de l'onde de choc est préférentiellement constitué par une pièce allongée 7 munie d'un moyen d'impact 8 à l'une de ses extrémités 9, ladite pièce allongée 7 étant montée de manière à pouvoir osciller autour d'une position d'équilibre autour d'un axe de rotation 10.

A titre d'exemple non limitatif, la pièce allongée 7 peut être réalisée en un fil métallique recouvert partiellement par une matière synthétique amortissante, par exemple, du Néoprène.

De manière essentielle, le moyen d'impact 8 du moyen 4 de formation de l'onde de choc présente une structure 11 en forme de V.

Cette structure 11 en forme de V va percuter la périphérie ou surface extérieure de l'oeuf 5 à analyser en plusieurs endroits différents de manière à obtenir une série de micro-chocs et une série d'échos résultants très nets et purs.

Dans le cadre d'un contrôle de qualité d'oeufs 5 de poule, la structure 11 en forme de V du moyen d'impact 8 possède avantageusement une masse comprise entre 2 g et 4 g, de préférence égale à environ 2,8 g. Ainsi, tout risque de détérioration, même minime de l'oeuf 5 à tester est totalement exclu.

Dans un mode de réalisation préférentiel, l'autre extrémité 9' de la pièce allongée 7 du moyen 4 de formation de l'onde de choc comporte au moins un élément magnétique 12 destiné à collaborer avec un autre élément magnétique 13 pour la formation du mouvement oscillatoire de la pièce allongée 7 autour d'une position d'équilibre et autour d'un axe de rotation 10.

Le mode de réalisation préférentiel représenté aux figures 2 et 3 montre que la pièce allongée 7 du moyen 4 de formation de l'onde de choc est pourvue, à son extrémité opposée 9' à celle comportant le moyen d'impact 8, d'un aimant (N-S) 12 attiré ou repoussé par un au moins autre aimant (N-S) 13 en mouvement.

Selon un mode de réalisation particulièrement préféré, la pièce allongée 7 du moyen 4 de formation de l'onde de choc comporte à son extrémité opposée 9' à celle comportant le moyen d'impact 8, un aimant 12 attiré ou repoussé par au moins un autre aimant 13 monté sur un axe 14 rotatif, par exemple l'axe 14 rotatif d'un moteur 15. A titre d'exemple particulièrement bien adapté, le moteur 15 utilisé peut être un moteur électrique possédant une vitesse de rotation réglable.

De cette manière, la rotation des pôles Nord-Sud (N-S) du ou des aimants 13 entraînés par le moteur 15 communique, de manière simple, fiable et efficace, un mouvement oscillatoire de haute fréquence à l'aimant 12 fixé à l'extrémité 9' de la pièce allongée 7 qui peut basculer autour de l'axe 10, et donc aussi au moyen d'impact 8 situé à l'autre extrémité 9. L'amplitude du mouvement de basculement de la pièce allongée 7 autour de l'axe 10 peut être avantageusement limitée par une ou plusieurs butées 16, par exemple des lamelles en Néoprène comme on peut le voir sur la figure 2.

Le moyen 6 d'acquisition de l'onde résultante de l'impact sur ledit produit 5 à analyser peut être tout capteur d'ondes, par exemple un capteur de sons tel qu'au moins un microphone 17 classique, placé à proximité, c'est-à-dire à environ 1 cm du moyen d'impact 8 qui se situe toujours au-dessus de l'oeuf 5.

Pour les machines à faible vitesse de tri, au moins deux microphones 17 à électret sont utilisés pour détecter les vibrations transmises par l'air. Pour les machines plus rapides on peut utiliser au moins deux capteurs de vibration à laser (non représentés). Les capteurs d'ondes sont placés de part et d'autre du moyen d'impact 8 provoquant la stimulation de l'oeuf 5 (cf. figure 3).

Les capteurs de vibrations sont mis en mode différentiel de manière à obtenir une meilleure résolution du signal et donc une meilleure définition de la qualité de la coquille. Comme déjà expliqué, le signal mesuré aboutit dans le microprocesseur central µPC qui fournit une valeur de la qualité de l'oeuf 5 et de sa coquille à l'aide d'algorithmes de logique floue.

Le dispositif d'analyse optique 3 par réflexion et transparence alternées est constitué par au moins un dispositif photonumérique rapide 18 du type caméra CCD combiné à au moins une source de lumière 19 travaillant à haute fréquence. A titre d'exemple non limitatif, une telle source de lumière 19 peut être un stroboscope, un flash conventionnel ou analogues. Dans un mode de réalisation préféré, le faisceau de lumière suit le mouvement des objets 5 à éclairer et se repositionne après la mesure pour l'objet 5 suivant.

Selon une variante particulièrement avantageuse, le dispositif 1 selon la présente invention est caractérisé en ce qu'il comporte au moins deux dispositifs d'analyse optique 3 synchronisés. Cette synchronisation se fait par l'intermédiaire d'un ensemble de moyens m pilotés par un microprocesseur µP comme symbolisé sur la figure 1.

De cette façon, on obtient pour chaque produit 5 analysé deux séries de clichés pris sous deux angles différents et qui permettent, de manière très avantageuse de photographier toute la surface, par exemple, d'un oeuf 5 et en particulier les régions des pôles d'un tel objet 5 sphérique ou ovoïde (cf. figure 4). On obtient avec la présence simultanée de deux capteurs d'images (dispositifs photonumériques rapides 18) disposés selon le mode préférentiel représenté sur la figure 4 un développement complet de toute la surface externe et interne de l'oeuf 5 analysé, ce qui n'est pas ou que difficilement possible avec un seul capteur.

En raison de l'alternance des modes de mesure, la moitié des clichés d'une série sera réalisée par transparence, l'autre moitié étant réalisée par réflexion.

De plus, l'utilisation de deux capteurs d'image ou dispositifs photonumériques rapides 18, sous la forme par exemple de deux caméras CCD, par oeuf 5 permet d'obtenir une meilleure résolution. Il est cependant également possible de n'utiliser qu'un seul capteur en combinaison avec un miroir courbe et un ensemble de lentilles adaptées (non représentés).

Chaque image de l'oeuf 5 est prise deux fois dans un intervalle très bref : la première image obtenue représentera l'oeuf 5 vu par transparence, l'oeuf 5 étant éclairé par la source de lumière 19 inférieure ; la seconde image représentera l'oeuf 5 vu par réflexion, l'oeuf étant éclairé par la source de lumière 19 supérieure (cf. figure 4). De manière avantageuse, chaque oeuf 5 est photographié au moins cinq fois.

La succession des images obtenues par transparence permet de déterminer la qualité de la coquille (recherche de piquage, d'étoile...) ainsi que la présence d'inclusions (taches restant dans la même zone pendant la rotation). La seconde série d'images permet de déterminer la qualité de coloration de la coquille ainsi que la recherche de taches de salissures sur l'oeuf 5.

De manière analogue au dispositif d'analyse ondulatoire 2, chaque dispositif d'analyse optique 3 effectue une série de relevés de signaux optiques qui sont transférés à un microprocesseur µP par l'intermédiaire d'un convertisseur analogique/numérique A/N. Un microprocesseur µP propre au dispositif d'analyse ondulatoire 2 établit une signature pour chaque échelon. Après un certain nombre d'échelons, il effectue un traitement de la série de signatures obtenue et les transforme en une série de paramètres correspondants. Ces paramètres sont ensuite transférés au microprocesseur central µPC qui les stocke, si nécessaire, dans une mémoire jusqu'au traitement final permettant, avec les paramètres obtenus par les autres microprocesseurs µP d'attribuer une note de qualité à l'oeuf 5 examiné.

Différents capteurs sont placés sur la chaîne d'entraînement des oeufs 5 ainsi que sur le dispositif d'analyse ondulatoire 2 mobile de manière à permettre la synchronisation de l'ensemble.

L'unité de traitement informatique des données est constituée par le microprocesseur central µPC qui corrèle les paramètres traités et effectue le classement de l'oeuf 5 analysé dans une catégorie de qualité prédéfinie par le producteur.

Le signal numérique de sortie S, vers un bus, de cette unité de comparaison permet ensuite la réalisation de toutes sortes d'opérations destinées à traduire le classement de la qualité de l'oeuf 5 (élimination, marquage, regroupement, stockage, conditionnement...).

Un capteur de position 20 (figure 1) déclenche et synchronise l'acquisition des données.

La présente invention fournit donc un procédé simple et peu onéreux à mettre en oeuvre permettant d'assurer un contrôle très efficace (fiabilité supérieure à 95 %) de la qualité de produits ou d'objets 5, en particulier d'oeufs 5 et notamment d'oeufs 5 de poule.

A titre d'exemples, et pour une application aux oeufs 5 tels que des oeufs 5 de poule, le procédé de la présente invention permet notamment d'évaluer les critères de qualité suivants : présence et importance de salissures et/ou d'inhomogénéités (aspect général, forme, couleur...), présence et importance de dégâts (micro-fissures, fissures, trous...) sur la coquille de l'oeuf 5 et présence et importance d'inclusions à l'intérieur de l'oeuf 5 (traces de sang, malformations, corps étrangers...).

## Revendications

1. Procédé non-destructif d'évaluation et de sélection de produits ou d'objets selon leurs qualités, **caractérisé en ce qu'**il comprend les étapes consistant à:
- opérer une série de mesures ondulatoires de manière à obtenir une série de signatures ondulatoires traitées pour fournir une première série de paramètres desdits produits ou objets (5) à analyser,
- opérer une série de mesures optiques de manière à obtenir une série de signatures optiques traitées pour fournir une seconde série de paramètres desdits produits ou objets (5) à analyser,
- corréler lesdites première et seconde séries de paramètres de façon à déterminer un facteur de qualité pour chaque produit ou objet (5) analysé, et,
- différencier les produits ou objets (5) analysés selon les facteurs de qualité obtenus.

2. Procédé selon la revendication 1, **caractérisé en ce que** les mesures ondulatoires sont effectuées en réalisant une succession d'impacts, à fréquence élevée, à différents endroits de la périphérie ou de la surface extérieure du produit ou de l'objet (5) à analyser, et à acquérir les ondes résultantes desdits impacts.

3. Procédé selon la revendication 1, **caractérisé en ce que** les mesures optiques sont réalisées par une série de relevés de signaux optiques alternativement par transparence et par réflexion.

4. Procédé selon l'une quelconque des revendications 1 à 3, **caractérisé en ce que** les produits ou objets (5) à analyser consistent en des oeufs, en particulier, en des oeufs de poule.

5. Dispositif pour la mise en oeuvre du procédé selon l'une quelconque des revendications 1 à 4, **caractérisé en ce qu'**il est constitué par :
- au moins un dispositif d'analyse ondulatoire (2),
- au moins un dispositif d'analyse optique (3), et,
- au moins un moyen de traitement informatique des données.

6. Dispositif selon la revendication 5, **caractérisé en ce qu'**il comporte au moins deux dispositifs d'analyse optique (3) synchronisés.

7. Dispositif selon la revendication 5 ou 6, **caractérisé en ce que** le dispositif d'analyse ondulatoire (2) comporte un premier moyen (4) de formation d'une onde de choc sur la surface extérieure du produit (5) à analyser ainsi qu'un second moyen (6) d'acquisition de l'onde résultante de l'impact sur ledit produit (5) à analyser.

8. Dispositif selon la revendication 7, **caractérisé en ce que** le moyen (4) de formation de l'onde de choc est constitué par une pièce allongée (7) munie d'un moyen d'impact (8) à l'une de des extrémités (9), ladite pièce allongée (7) étant montée de manière à pouvoir osciller autour d'une position d'équilibre autour d'un axe de rotation (10).

9. Dispositif selon la revendication 7 ou 8, **caractérisé en ce que** le moyen d'impact (8) du moyen (4) de formation de l'onde de choc présente une structure (11) en forme de V.

10. Dispositif selon la revendication 9, **caractérisé en ce que** la structure (11) en forme de V du moyen d'impact (8) possède une masse comprise entre 2 g et 4 g, de préférence égale à environ 2,8 g.

11. Dispositif selon l'une quelconque des revendications 7 à 10, **caractérisé en ce que** l'autre extrémité (9') de la pièce allongée (7) du moyen (4) de formation de l'onde de choc comporte au moins un élément magnétique (12) destiné à collaborer avec un autre élément magnétique (13) pour la formation du mouvement oscillatoire de la pièce allongée (7) autour d'une position d'équilibre et autour d'un axe de rotation (10).

12. Dispositif selon la revendication 11, **caractérisé en ce que** la pièce allongée (7) du moyen (4) de formation de l'onde de choc est pourvue à son extrémité opposée (9') à celle comportant le moyen d'impact (8), d'un aimant (N-S) (12) attiré ou repoussé par au moins un autre aimant (N-S) (13) en mouvement.

13. Dispositif selon la revendication 12, **caractérisé en ce que** la pièce allongée (7) du moyen (4) de formation de l'onde de choc comporte à son extrémité opposée (9') à celle comportant le moyen d'impact (8), un aimant (12) attiré ou repoussé par au moins un autre aimant (13) monté sur un axe (14) rotatif, par exemple sur l'axe (14) rotatif d'un moteur (15).

14. Dispositif selon la revendication 5 ou 6, **caractérisé en ce que** le dispositif d'analyse optique (3) par réflexion et transparence alternées est constitué par au moins un dispositif photonumérique (18) rapide, du type caméra CCD, combiné à au moins une source de lumière travaillant à haute fréquence (19), par exemple un stroboscope.

## Patentansprüche

1. Zerstörungsfreies Verfahren zum Bewerten und Auswählen von Produkten und Gegenständen ihren Eigenschaften entsprechend, **dadurch gekennzeichnet, daß** es die folgenden Schritte umfaßt:
- Durchführen einer Reihe von Wellenmessungen, um eine Reihe von Wellensignaturen zu erhalten, die verarbeitet werden, um eine erste Parameterreihe für diese zu analysierenden Produkte oder Gegenstände (5) zu erhalten,
- Durchführen einer Reihe von optischen Messungen, um eine Reihe von optischen Signaturen zu erhalten, die verarbeitet werden, um eine zweite Parameterreihe für diese zu analysierenden Produkte oder Gegenstände (5) zu erhalten,
- Korrelieren der ersten und zweiten Parameterreihe, um einen Qualitätsfaktor für jedes Produkt oder jeden Gegenstand (5) zu erhalten, das oder der analysiert wird, und
Differenzieren der analysierten Produkte oder Gegenstände (5) entsprechend den erhaltenen Qualitätsfaktoren.

2. Verfahren gemäß Anspruch 1, **dadurch gekennzeichnet, daß** die Wellenmessungen durchgeführt werden, indem eine Folge von Stößen mit hoher Frequenz an verschiedenen Stellen am Umfang oder an der Außenfläche des zu analysierenden Produkts oder Gegenstands (5) durchgeführt wird und indem die Wellen erfaßt werden, die aus diesen Stößen resultieren.

3. Verfahren gemäß Anspruch 1, **dadurch gekennzeichnet, daß** die optischen Messungen durchgeführt werden, indem durch eine Reihe von optischen Signalen abwechselnd die Transparenz und die Reflexion gemessen wird.

4. Verfahren gemäß einem der Ansprüche 1 bis 3, **dadurch gekennzeichnet, daß** die zu analysierenden Produkte oder Gegenstände (5) Eier, insbesondere Hühnereier, sind.

5. Vorrichtung zur Durchführung des Verfahrens gemäß einem der Ansprüche 1 bis 4, **dadurch gekennzeichnet, daß** sie folgendes umfaßt:
- mindestens eine Wellenanalysevorrichtung (2),
- mindestens eine optische Analysevorrichtung (3), und
- mindestens ein Datenverarbeitungsmittel.

6. Vorrichtung gemäß Anspruch 5, **dadurch gekennzeichnet, daß** sie mindestens zwei synchronisierte optische Analysevorrichtungen (3) umfaßt.

7. Vorrichtung gemäß Anspruch 5 oder 6, **dadurch gekennzeichnet, daß** die Wellenanalysevorrichtung (2) ein erstes Mittel (4) zum Erzeugen einer Stoßwelle auf der Außenfläche des zu analysierenden Produkts (5) umfaßt, sowie ein zweites Mittel (6) zum Erfassen der Welle, die aus dem Stoß auf das zu analysierende Produkt (5) resultiert.

8. Vorrichtung gemäß Anspruch 7, **dadurch gekennzeichnet, daß** das Mittel (4) zum Erzeugen der Stoßwelle ein längliches Teil (7) umfaßt, das an einem seiner Enden (9) mit einem Stoßmittel (8) versehen ist, wobei dieses längliche Teil (7) so befestigt ist, daß es um eine Drehachse (10) um eine Gleichgewichtslage herum schwingen kann.

9. Vorrichtung gemäß Anspruch 7 oder 8, **dadurch gekennzeichnet, daß** das Stoßmittel (8) des Mittels (4) zum Erzeugen der Stoßwelle einen V-förmigen Aufbau (11) aufweist.

10. Vorrichtung gemäß Anspruch 9, **dadurch gekennzeichnet, daß** der V-förmige Aufbau (11) des Stoßmittels (8) ein Gewicht aufweist, das zwischen 2 g und 4 g liegt und vorzugsweise 2,8 g entspricht.

11. Vorrichtung gemäß einem der Ansprüche 7 bis 10, **dadurch gekennzeichnet, daß** das andere Ende (9') des länglichen Teils (7) des Mittels (4) zum Erzeugen der Stoßwelle mindestens ein magnetisches Element (12) aufweist, das vorgesehen ist, um mit einem anderen magnetischen Element (13) zusammenzuwirken, um die Schwingbewegung des länglichen Teils (7) um eine Gleichgewichtslage und um eine Drehachse (10) zu bewirken.

12. Vorrichtung gemäß Anspruch 11, **dadurch gekennzeichnet, daß** das längliche Teil (7) des Mittels (4) zum Erzeugen der Stoßwelle an seinem Ende (9'), das dem mit dem Stoßmittel (8) entgegengesetzt ist, mit einem Magneten (N-S) (12) versehen ist, der von mindestens einem anderen Magneten (13) (N-S), der in Bewegung ist, angezogen oder abgestoßen wird.

13. Vorrichtung gemäß Anspruch 12, **dadurch gekennzeichnet, daß** das längliche Teil (7) des Mittels (4) zum Erzeugen der Stoßwelle an seinem Ende (9'), das dem mit dem Stoßmittel (8) entgegengesetzt ist, einen Magneten (12) umfaßt, der von mindestens einem anderen Magneten (13) angezogen oder abgestoßen wird, der an einer Rotationsachse (14), zum Beispiel an der Rotationsachse (14) eines Motors (15) befestigt ist.

14. Vorrichtung gemäß Anspruch 5 oder 6, **dadurch gekennzeichnet, daß** die optische Analysevorrichtung (3) zur abwechselnden Reflexions- und Transparenzmessung mindestens eine schnelle digitale Kamera vom Typ CCD-Kamera umfaßt, die mit mindestens einer Lichtquelle kombiniert ist, die mit Hochfrequenz (19) arbeitet, wie zum Beispiel ein Stroboskop.

## Claims

1. Method for non-destructive assessment and selection of products or objects according to their qualities, **characterised in that** it comprises steps which consist in:
- performing a series of oscillatory measurements so as to obtain a series of oscillatory signatures processed to supply a first series of parameters of said products or objects (5) to be analysed,
- performing a series of optical measurements so as to obtain a series of optical signatures processed to supply a second series of parameters of said products or objects (5) to be analysed,
- correlating said first and second series of parameters so as to determine a quality factor for each analysed product or object (5), and,
- distinguishing between the analysed products or objects (5) according to the quality factors obtained.

2. Method according to claim 1, **characterised in that** the oscillatory measurements are carried out by performing a series of high-frequency impacts at different points of the periphery or extemal surface of the product or object (5) to be analysed and receiving the waves resulting from said impacts.

3. Method according to claim 1, **characterised in that** the optical measurements are made by a series of optical signal readings, alternately by transparency and reflection.

4. Method according to any one of claims I to 3, **characterised in that** the products or objects (5) to be analysed consist of eggs, in particular, hens' eggs.

5. Device for implementing the method according to any one of claims 1 to 4, **characterised in that** it consists of
- at least one oscillatory analysis device (2)
- at least one optical analysis device (3) and
- at least one data processing means.

6. Device according to claim 5, **characterised in that** it comprises at least two synchronised optical analysis devices (3).

7. Device according to claim 5 or 6, **characterised in that** the oscillatory analysis device (2) comprises a first means (4) for producing a shock wave on the external surface of the product (5) to be analysed, in addition to a second means (6) for receiving the wave resulting from the impact on said product (5) to be analysed.

8. Device according to claim 7, **characterised in that** the means (4) for producing the shock wave comprises an elongated part (7) having impact means (8) at one end (9), said elongated part (7) being mounted so as to be able to oscillate about an equilibrium position about an axis of rotation (10).

9. Device according to claim 7 or 8, **characterised in that** the impact means (8) for the means (4) for producing the shock wave has a V-shaped structure (11).

10. Device according to claim 9, **characterised in that** the V-shaped structure (11) of the impact means (8) has a total weight of between 2 g and 4 g, preferably approximately 2.8 g.

11. Device according to any one of claims 7 to 10, **characterised in that** the other end (9') of the elongated part (7) of the means (4) for producing the shock wave comprises at least one magnetic element (12) designed to work in conjunction with another magnetic element (13) to produce the oscillating movement of the elongated part (7) about an equilibrium position and about an axis of rotation (10).

12. Device according to claim 11, **characterised in that** the elongated part (7) of the means (4) for producing the shock wave is provided, on the opposite end (9') to that having impact means (8), with a (N-S) magnet (12) attracted to or repelled by at least one other (N-S) moving magnet (13).

13. Device according to claim 12, **characterised in that** the elongated part (7) of the means (4) for producing the shock wave comprises on the opposite end (9') to that having impact means (8) a magnet (12) attracted to or repelled by at least one other magnet (13) mounted on a revolving shaft (14), for example on the revolving shaft of a motor (15).

14. Device according to claim 5 or 6, **characterised in that** the optical analysis device (3), by alternate reflection and transparency, consists of at least one high-speed digital photographic device (18), of the CCD camera type, combined with at least one high-frequency light source (19), for example a stroboscope.
